# EUROPÄISCHE PATENTSCHRIFT

(11) **EP 1 878 389 B1**
(45) Veröffentlichungstag und Bekanntmachung des Hinweises auf die Patenterteilung: **09.12.2009**
(21) Anmeldenummer: 07011531.6
(22) Anmeldetag: 13.06.2007
(51) Int. Cl.: A61B 17/00

(54) **Vorrichtung zur Positionierung eines Werkzeuges**
Device for positioning a tool
Dispositif destiné au positionnement d'un outil

(30) Priorität: 04.07.2006 DE 102006030689
(43) Veröffentlichungstag der Anmeldung: 16.01.2008
(73) Patentinhaber: Helmut-Schmidt-Universität/ Universität der Bundeswehr Hamburg, 22043 Hamburg (DE)
(72) Erfinder: Faber, Axel, 22083 Hamburg (DE)
(74) Vertreter: Klickow, Hans-Henning

(56) Entgegenhaltungen:
- WO-A-01/28437
- WO-A-99/12485
- WO-A-2004/016196
- GB-A- 986 134
- US-A- 5 690 660
- US-A- 6 018 094

## Beschreibung

Die Erfindung betrifft eine Vorrichtung zur Positionierung eines Werkzeuges zur Bearbeitung von Knochen, die einen Schaft aufweist, der im Bereich seines einen Endes über ein reversibel verformbares Tragelement mit dem Werkzeug verbunden ist und bei dem ein mit einem Abstand zu diesem Ende angeordneter Bereich zur Einleitung von Positionierkräften vorgesehen ist, und bei der der Schaft mindestens bereichsweise als eine gekrümmte Führung ausgebildet ist, entlang derer das Tragelement geführt ist.

Derartige Vorrichtungen werden beispielsweise verwendet, um spanabhebende verfahren wie Fräsen, Bohren oder Schleifen durchzuführen. Die Vorrichtungen sind in der Regel gut dafür geeignet, im Bereich von gut zugänglichen Bereichen des Werkstückes eingesetzt zu werden, eine Verwendung an unzugänglichen Stellen oder eine Umgehung von Hindernissen führt jedoch in der Regel zu Problemen.

Eine besondere Anwendungsproblematik besteht im Bereich der Endoprothetik und insbesondere im Bereich der Hüftendoprothetik. Zur Bearbeitung von unzugänglichen Stellen werden hier insbesondere bei der minimalinvasiven Hüftchirurgie speziell gebogene Räumwerkzeuge in der Ausbildung eines Raspels verwendet. Die mit Hilfe dieser Werkzeuge erzeugten Kavitäten sind hinsichtlich ihrer Fertigungsgenauigkeit stark von der Erfahrung und den Fähigkeiten des Chirurgen abhängig.

Aus der US 5,690,660 A ist eine Schneideinrichtung für Knochen bekannt, bei der sich innerhalb einer abschnittweise gekrümmt verlaufenden Führung eine flexible rotierende Antriebswelle für das Schneidwerkzeug erstreckt. Die Antriebswelle und das Schneidwerkzeug können nicht in einer Längsrichtung aus der Führung herausgeschoben werden.

Aus der WO 2004/016196 A ist ebenfalls ein medizintechnisches Werkzeug bekannt, bei dem entlang einer gekrümmt verlaufenden Führung ein Tragelement positionierbar ist. Das Tragelement kann in einer Längsrichtung aus der rohrartigen Führung herausgeschoben werden.

In der WO 01/28437 A wird eine weitere medizintechnische Einrichtung beschrieben, bei der sich innerhalb einer rohrartigen Führung ein Tragelement für ein rotatorisch angetriebenes Werkzeug befindet.

Aufgabe der vorliegenden Erfindung ist es, eine Vorrichtung der einleitend genannten Art derart zu konstruieren, daß eine Bearbeitung von Werkstücken an unzugänglichen Stellen mit hoher Genauigkeit unterstützt wird.

Diese Aufgabe wird erfindungsgemäß dadurch gelöst, daß das Tragelement aus einer Mehrzahl beweglich relativ zueinander angeordneter Gliederelemente ausgebildet ist und daß die Gliederelemente mindestens außerhalb der Führung relativ zueinander stabilisiert sind, und daß das Tragelement insgesamt im stabilisierten Zustand der Gliederelemente relativ zueinander relativ zur Führung verdrehsicher geführt ist.

Als vorteilhaft erweist es sich, daß entlang der Führung als Tragelement eine Mehrzahl beweglicher relativ zueinander angeordneter Gliederelemente geführt sind und daß die Gliederelemente mindestens außerhalb der Führung relativ zueinander stabilisiert sind.

Durch die Ausbildung des Schaftes als Führung für das Tragelement oder für eine Mehrzahl von Gliederelementen und die Stabilisierung der Gliederelemente relativ zueinander außerhalb der Führung ist es möglich, die Gliederelemente um Hindernisse herum oder in den Bereich von unzugänglichen Stellen zu bewegen und hierdurch ein am Ende der Glieder elemente angeordnetes Werkzeug in optimaler Weise zu positionieren.

Bei einer Anwendung im Bereich der Hüftendoprothetik läßt sich durch die Anwendung der Erfindung die Invasivität bei einem Fräseinsatz am Hüftknochen erheblich reduzieren. Es kann insbesondere eine Verkürzung des chirurgischen Muskelschnittes erfolgen und hierdurch eine Reduzierung von Muskel- und Nervenschäden erreicht werden. In kosmetischer Hinsicht kann ebenfalls eine deutlich verkürzte sichtbare Narbe an der Hautoberfläche erreicht werden.

Weitere Anwendungsgebiete sind das Polieren von Strömungskanälen, insbesondere bei Motoren und das Fräsen oder Schleifen bei mechanischen Bearbeitungen.

Eine typische Anwendung ist dadurch definiert, daß das Werkzeug zur Durchführung von mechanischen Bearbeitungen ausgebildet ist. Es können auch Laserwerkzeuge, Wasserstrahlwerkzeuge und/oder endoskopische Werkzeuge verwendet werden.

Ein Materialabtrag wird insbesondere dadurch unterstützt, daß das Werkzeug mit einem Antriebsmotor verbunden ist.

Zur Verwendung von rotierenden Werkzeugen erweist es sich als vorteilhaft, daß sich eine Antriebswelle für das Werkzeug entlang der Führung erstreckt.

Ebenfalls ist daran gedacht, daß sich eine Antriebswelle für das Werkzeug innerhalb einer rohrartigen Umhüllung erstreckt.

Positionsveränderungen während der Durchführung von Bearbeitungsvorgängen werden dadurch unterstützt, daß die Gliederelemente mit einer Vorschubeinrichtung gekoppelt sind.

Ein großer Freiraum bei der Vorgabe von Bewegungen wird dadurch gewährleistet, daß die Vorschubeinrichtung zur Einbringung von Zug- und Druckkräften ausgebildet ist.

Ein weiteres Anwendungsgebiet besteht darin, daß die Vorrichtung zur Verwendung im medizinischen Bereich ausgebildet ist.

Insbesondere ist daran gedacht, daß das Werkzeug zur Herstellung von Kavitäten für Endoprothesen ausgebildet ist.

Eine vorteilhafte Anwendung der Erfindung kann auch dadurch erfolgen, daß eine Ausbildung zur Herstellung von Kavitäten für Hüftgelenkendoprothesen vorliegt.

Vergleichsweise große Positionierkräfte lassen sich dadurch erzeugen, daß die Vorschubeinrichtung einen Linearantrieb aufweist. Dieser kann beispielsweise als ein Spindelantrieb ausgebildet sein.

Eine ausreichend große Steifigkeit der Gliederelemente trotz einer beweglichen Verbindung kann dadurch erreicht werden, daß die Gliederelemente relativ zueinander von einem Federstahldraht verspannt sind.

In den Zeichnungen sind Ausführungsbeispiele der Erfindung dargestellt. Es zeigen:
- Fig. 1: eine schematische Darstellung zur Veranschaulichung einer gebogen verlaufenden Führung und von Gliederelementen, die bereichsweise aus der Führung herausragen,
- Fig. 2: eine Draufsicht auf eine Ausführungsform von Gliederelementen,
- Fig. 3: die Gliederelemente gemäß Blickrichtung III in Fig. 2,
- Fig. 4: die Gliederelemente gemäß Blickrichtung IV in Fig. 3,
- Fig. 5: eine Ausführungsform einer rohrartigen Führung, aus der Gliederelemente herausragen, die einen Fräskopf tragen,
- Fig. 6: die Anordnung gemäß Fig. 5 nach einem Ausfahren diverser Gliederelemente aus der Führung,
- Fig. 7: eine Seitenansicht einer Vorrichtung mit rohrartiger Führung,
- Fig. 8: ein Längsschnitt gemäß Schnittlinie VIII-VIII in Fig. 7,
- Fig. 9: einen weiteren Längsschnitt zur Veranschaulichung einer Anordnung der Gliederelemente im gebogenen Verlauf der Führung,
- Fig. 10: eine Ausführungsform zur Verwendung im Bereich der Hüftchirurgie,
- Fig. 11: die Vorrichtung in Fig. 10 gemäß Blickrichtung XI und
- Fig. 12: die Vorrichtung in Fig. 10 gemäß Blickrichtung XII.

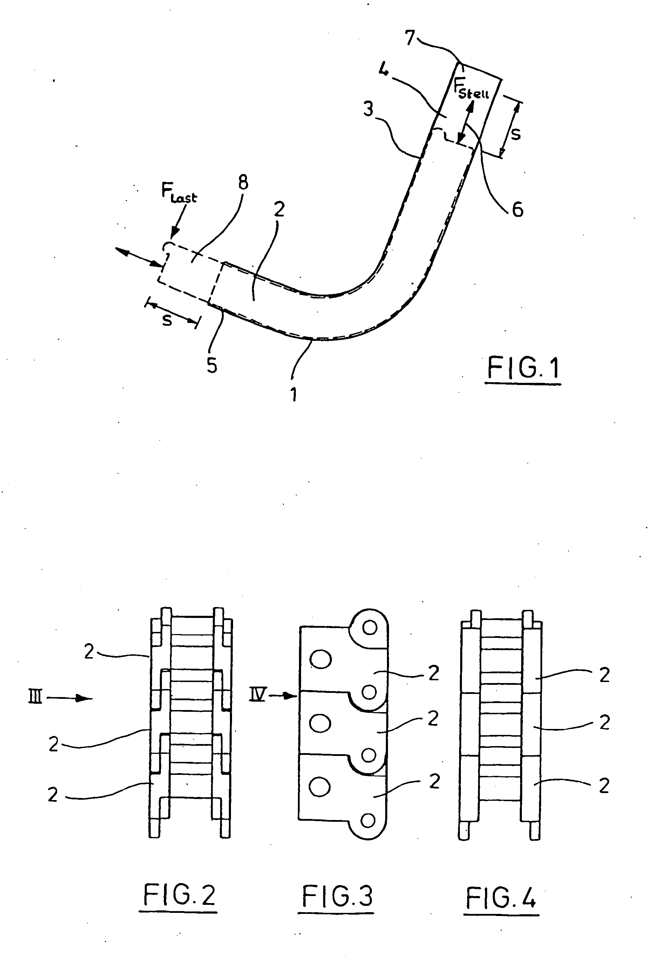

Gemäß der Darstellung in Fig. 1 wird das Funktionsprinzip der vorliegenden Erfindung veranschaulicht. Entlang einer Führung (1), die mindestens bereichsweise gebogen verläuft, sind Gliederelemente (2) positionierbar. Gemäß der Ausführungsform in Fig. 1 ist die Führung in Fig. 1 als ein Schaft (3) ausgebildet. Der Schaft (3) kann als ein Hohlkörper ausgeführt sein, der einen Innenraum (4) aufweist. Im Innenraum (4) sind die Gliederelemente (2) angeordnet. Statt der Gliederelemente können auch beliebige andere reversibel verformbare Tragelemente zum Einsatz kommen. Gedacht ist beispielsweise an elastische oder elastomere Stäbe sowie an Federstahlelemente.

Die Gliederelemente (2) sind derart miteinander verbunden, daß diese stabil aus einem Ende (5) des Schaftes (3) herausragen. In Abhängigkeit von der Einwirkung einer Stellkraft (6), die die Gliederelemente (2) im Bereich eines Endes (7) des Schaftes (3) beaufschlagt, das dem Ende (5) gegenüberliegt, ist es möglich, die Gliederelemente (2) vorgebbar weit aus dem Schaft (3) herauszuschieben. Gemäß der Darstellung in Fig. 1 ist ein Überstand (8) einer Länge S realisiert.

Fig. 2 zeigt eine Mehrzahl von kettenartig ineinandergreifender Gliederelemente (2). Die Gliederelemente (2) sind relativ zueinander sowohl beweglich als auch fixierbar angeordnet. Die Beweglichkeit wird bei einem Überschreiten einer Mindestquerkraft erreicht. Ohne eine derartige Querkraft verbleiben die Gliederelemente (2) relativ zueinander in einer Grundposition. Die Stabilisierung kann beispielsweise durch elastische oder federnde Rastelemente, allgemeiner Rastelemente, elastische Rastelemente oder durch eine Verspannung realisiert sein.

Fig. 3 zeigt die Anordnung in Fig. 2 gemäß Blickrichtung III. Es ist zu erkennen, daß die Gliederelemente bei dieser Ausführungsform in einer Bewegungsrichtung relativ zueinander verschwenkbar angeordnet sind. Hierdurch können die Gliederelemente (2) relativ zu einer vorgegebenen Krümmungsbahn geführt werden.

Fig. 4 zeigt die Anordnung der Gliederelemente aus Fig. 3 gemäß Blickrichtung IV. Auch hier ist die Anordnung der einzelnen Gliederelemente (2) relativ zueinander veranschaulicht.

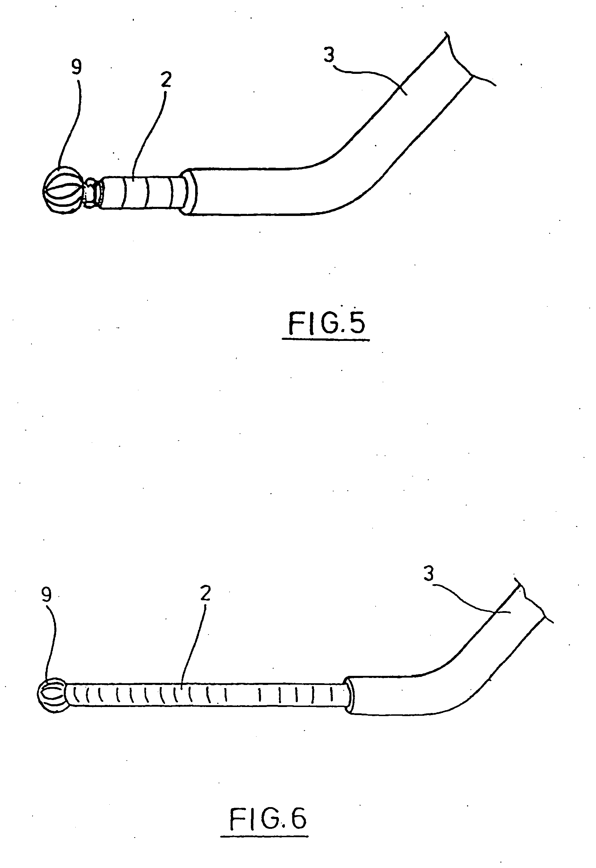

Fig. 5 zeigt eine Ausführungsform, bei der im Bereich des aus dem Schaft (3) herausragenden Teiles der Gliederelemente (2) ein Werkzeug (9) angeordnet ist. Das Werkzeug (9) kann beispielsweise als ein Fräskopf realisiert sein.

Gemäß der Darstellung in Fig. 6 ragen die Gliederelemente (2) gegenüber der Darstellung in Fig. 5 deutlich weiter aus dem Schaft (3) heraus. Zu erkennen ist insbesondere die sehr stabile und lineare Anordnung der einzelnen Gliederelemente (2) relativ zueinander.

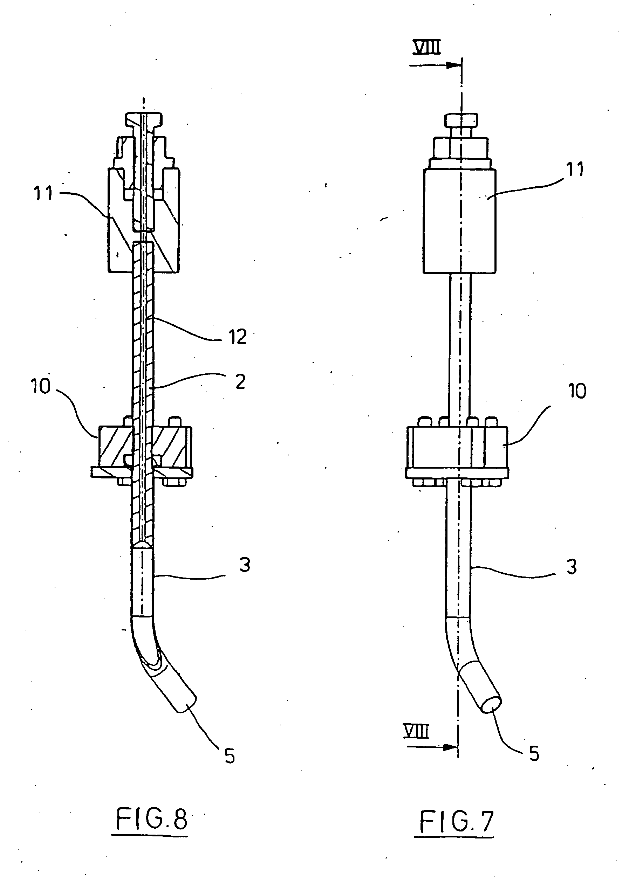

Fig. 7 veranschaulicht den Aufbau des Schaftes (3) sowie zugeordneter Bauelemente in stärkerer Detailliertheit. Zur Positionierung des Schaftes (3) wird eine Verstelleinrichtung (10) verwendet, die beispielsweise mit einem Spindelantrieb ausgestattet ist. Ebenfalls ist am Schaft (3) eine Halteeinrichtung (11) angeordnet. Die Halteeinrichtung (11) kann beispielsweise als Spanneinrichtung für ein in Fig. 8 dargestelltes Verbindungselement (12) der Gliederelemente (2) verwendet werden. Eine Realisierung des Verbindungselementes (12) kann beispielsweise als Federstahldraht, Stahlband, Seilzug oder Kette erfolgen.

Fig. 8 veranschaulicht ebenfalls den Verlauf des Verbindungselementes (12) durch die Gliederelemente (2) hindurch. Aufgrund der verspannung der Gliederelemente (2) relativ zueinander unter Verwendung des Verbindungselementes (12) können die Gliederelemente (2) vergleichsweise starr aus dem Ende (5) des Schaftes (3) herausragen.

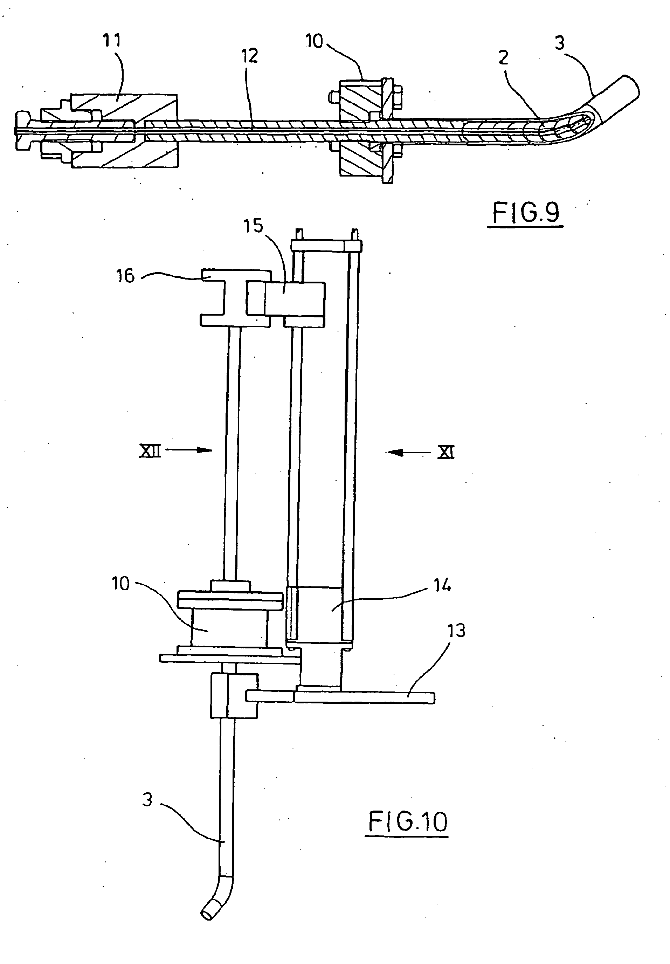

Fig. 9 zeigt die Anordnung einer Mehrzahl von Gliederelementen (2) im gebogenen Bereich des Schaftes (2) sowie den Verlauf des Verbindungselementes (12) durch die Gliederelemente hindurch. Es ist insbesondere zu erkennen, wie die Anordnung der Gliederelemente (2) sich an den gebogenen Verlauf des Schaftes (3) anpaßt.

Fig. 10 zeigt in einer Seitenansicht eine Ausführungsform zur Anwendung im Bereich der Hüftchirurgie. Diese Ausführungsform ist insbesondere auch dafür geeignet, in Kombination mit einem Operationsroboter verwendet zu werden. Hierzu ist ein Flansch (13) zur Ankopplung an einen nicht dargestellten Roboterarm vorgesehen. Zur Höhenpositionierung ist beispielsweise ein Schrittmotor (14) verwendet. Eine Spanneinrichtung (15) greift in einen Flansch (16) für einen Fräsantrieb ein.

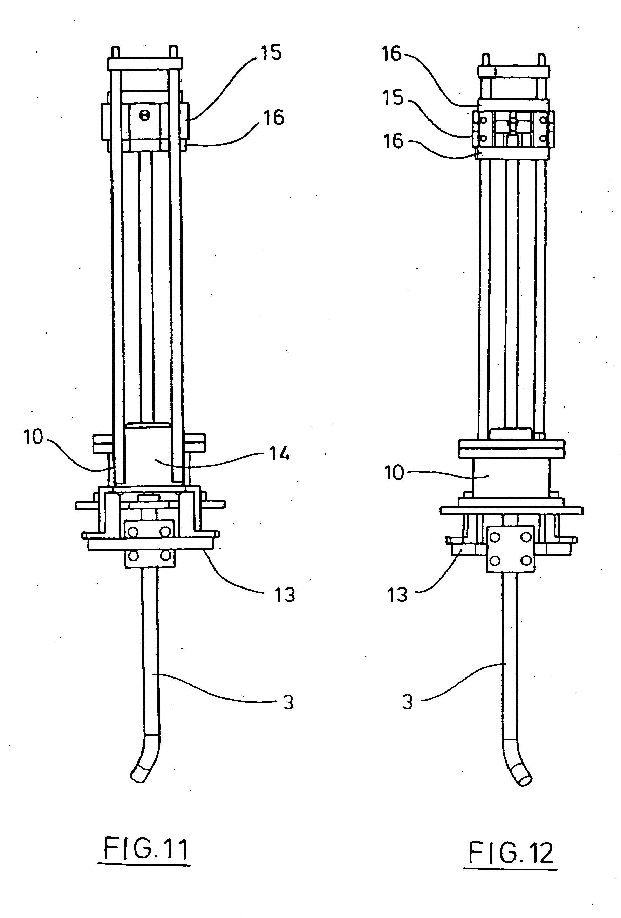

Fig. 11 und Fig. 12 veranschaulichen in zwei weiteren Ansichten die räumliche Anordnung des Schaftes (3), der Verstelleinrichtung (10) und des Flansches (16) relativ zum Schrittmotor (14) sowie zur Spanneinrichtung (15).

Aufgrund der Verspannung der Gliederelemente (12) relativ zueinander bzw. aufgrund der Fixierbarkeit der Gliederelemente (2) relativ zueinander erfolgt durch den aus dem Schaft (3) herausragenden Bereich der Gliederelemente (2) bezüglich der Positionierung des Werkzeuges (9) quasi eine Verlängerung des Schaftes (3). Unabhängig von der Länge des aus dem Schaft (3) herausragenden Teiles der Gliederelemente (2) wird eine sehr starre und zuverlässig positionierbare Anordnung des Werkzeuges (9) gewährleistet, so daß die erforderlichen Materialbearbeitungen mit hoher Präzision durchgeführt werden können.

Bei einer Anwendung der erfindungsgemäßen Vorrichtung im Bereich der Hüftchirurgie kann ein Einsatz der Vorrichtung mit den nachfolgend erläuterten Einzelschritten erfolgen. Der Chirurg erzeugt zunächst einen vergleichsweise klein dimensionierten Zugang mit einer Schnittlänge von etwa 6 bis 7 Zentimetern. Die Schnittlänge ist somit um den Faktor 4 geringer als bei einer konventionellen operativen Arbeitsweise. Anschließend wird das Bearbeitungsmedium durch die Wundöffnung hindurch eingeführt. Die Wundöffnung wird hierbei unter Verwendung eines Wundspreizers aufgehalten und stellt einen nutzbaren Durchmesser von etwa vier bis sechs Zentimetern bereit.

Der zu bearbeitende Oberschenkelknochen befindet sich in der Regel in einer Tiefe von etwa sieben bis acht Zentimetern. Das Bearbeitungsmedium muß durch die Wundöffnung hindurch in diese Tiefe gelangen und dann in axialer Richtung des Oberschenkelknochens die erforderliche Kavität für die Endoprothese erzeugen. Eine typische Paßform für die Prothese weist eine Länge von 100 bis 250 Millimeter auf. Durch die Verwendung des gebogenen Schaftes (3) kann sich der zur Knochenbearbeitung eingesetzte Endpunkt, der die Positionierung des Werkzeuges (9) wiedergibt, verlängern oder verkürzen, ohne daß die Position des gekrümmten Teiles des Schaftes (3) verändert wird.

Bei einer Krafteinleitung in die Gliederelemente (2) zur Durchführung der erforderlichen Stabilisierung kann die Krafteinleitung beispielsweise zentrisch oder nicht zentrisch erfolgen. Bei der in Fig. 9 veranschaulichten zentrischen Krafteinleitung kann sich das krafterzeugende System entsprechend der zeichnerischen Darstellung im Mittelpunkt der einzelnen Gliederelemente (2) befinden. Es ist aber auch möglich, das krafterzeugende System außerhalb der Gliederelemente (2) oder versetzt zu einer Mittellinie anzuordnen. Bei einer zentrischen Krafteinleitung ergibt sich als Vorteil eine in radialer Richtung über den Querschnitt nahezu gleich große Kraftbelastung. Alternativ zur Verwendung eines einzelnen krafterzeugenden Systems ist es auch möglich, eine mehrfache Krafteinleitung vorzusehen.

Ein Antrieb des Werkzeuges (9) kann beispielsweise unter Verwendung einer biegsamen Welle erfolgen, die in einem Kanal angeordnet ist. Für das Werkzeug (9) wird vorzugsweise eine Mikrolagerung verwendet. Als Antrieb, beispielsweise als Spindeltrieb, wird ein Stellmotor verwendet, der üblicherweise als Elektromotor realisiert ist. Eine hochgenaue Verstellung wird durch die Vorschaltung eines Getriebes unterstützt.

Die verdrehsichere Führung des Tragelementes entlang des Schaftes (3) unterstützt die bei der Bearbeitung von Knochen, insbesondere bei einer Fräsbearbeitung, erforderliche hohe Krafteinbringung in das zu bearbeitende Knochenmaterial. Es wird hierdurch eine relativ starre Grundkonstruktion bereitgestellt, die eine Verwendung von verriegelungen oder Entriegelungen vermeidet. Das aus dem Schaft (3) herausstehende Teil des Tragelementes, beispielsweise die gegeneinander stabilisierten Gliederelemente (2), sind im wesentlichen durch ihre Anordnung relativ starr und unterstützen hierdurch eine hochgenaue Positionierung des Werkzeuges (9). Es wird insbesondere auch ein Antrieb des Werkzeuges (9) mit den für eine Knochenbearbeitung typischen Drehzahlen im Bereich von 10.000 bis 50.000 Umdrehungen/Minute eines Fräskopfes des Werkzeuges (9) unterstützt. Die hohe Positioniergenauigkeit wird insbesondere auch durch eine spielfreie Anordnung der einzelnen Bauelemente relativ zueinander unterstützt.

## Patentansprüche

1. Vorrichtung zur Positionierung eines Werkzeuges (9) zur Bearbeitung von Knochen, die einen Schaft (3) aufweist, der im Bereich seines einen Endes (5) über ein reversibel verformbares Tragelement mit dem Werkzeug (9) verbunden ist und bei dem ein mit einem Abstand zu diesem Ende (5) angeordneter Bereich zur Einleitung von Positionierkräften vorgesehen ist, und bei der der Schaft (3) mindestens bereichsweise als eine gekrümmte Führung (1) ausgebildet ist, entlang derer das Tragelement geführt ist, **dadurch gekennzeichnet, daß** das Tragelement aus einer Mehrzahl beweglich relativ zueinander angeordneter Gliederelemente (2) ausgebildet ist und **daß** die Gliederelemente (2) mindestens außerhalb der Führung (1) relativ zueinander stabilisiert sind, und daß das Tragelement insgesamt im stabilisierten zustand der Gliederelemente (2) relativ zueinander relativ zur Führung (1) verdrehsicher geführt ist.

2. Vorrichtung nach Anspruch 1, **dadurch gekennzeichnet, daß** das Tragelement als ein elastisch verformbarer Stab ausgebildet ist.

3. Vorrichtung nach Anspruch 1, **dadurch gekennzeichnet, daß** das Tragelement als ein elastisch verformbarer Federstahl ausgebildet ist.

4. Vorrichtung nach einem der Ansprüche 1 bis 3, **dadurch gekennzeichnet, daß** das Werkzeug (9) zur Durchführung von mechanischen Bearbeitungen ausgebildet ist.

5. Vorrichtung nach Anspruch 1 bis 4, **dadurch gekennzeichnet, daß** das Werkzeug (9) mit einem Antriebsmotor verbunden ist.

6. Vorrichtung nach einem der Ansprüche 1 bis 5, **dadurch gekennzeichnet, daß** sich eine Antriebswelle für das Werkzeug (9) entlang der Führung erstreckt.

7. Vorrichtung nach einem der Ansprüche 1 bis 6, **dadurch gekennzeichnet, daß** sich eine Antriebswelle für das Werkzeug (9) innerhalb einer rohrartigen Umhüllung erstreckt.

8. Vorrichtung nach einem der Ansprüche 1 bis 7, **dadurch gekennzeichnet, daß** das Tragelement mit einer Vorschubeinrichtung gekoppelt ist.

9. Vorrichtung nach Anspruch 8, **dadurch gekennzeichnet, daß** die Vorschubeinrichtung zur Einbringung von Zug- und Druckkräften ausgebildet ist.

10. Vorrichtung nach einem der Ansprüche 1 bis 9, **dadurch gekennzeichnet, daß** die Vorrichtung zur Verwendung im medizinischen Bereich ausgebildet ist.

11. Vorrichtung nach einem der Ansprüche 1 bis 10, **dadurch gekennzeichnet, daß** das Werkzeug (9) zur Herstellung von Kavitäten für Endoprothesen ausgebildet ist.

12. Vorrichtung nach Anspruch 11, **dadurch gekennzeichnet, daß** eine Ausbildung zur Herstellung von Kavitäten für Hüftgelenkendoprothesen vorliegt.

13. Vorrichtung nach einem der Ansprüche 1 bis 12, **dadurch gekennzeichnet, daß** die Vorschubeinrichtung einen Linearantrieb aufweist.

14. Vorrichtung nach einem der Ansprüche 1 bis 13, **dadurch gekennzeichnet, daß** die Gliederelemente (2) relativ zueinander von einem Federstahldraht verspannt sind.

## Claims

1. A device for positioning a tool (9) for machining bone, which device comprises a shank (3), which in the area of one (5) of its ends is joined via a reversibly deformable carrier member to the tool (9) and in which a zone arranged at a distance from said end (5) is provided for introducing positioning forces, and in which device the shank (3) is constructed at least in places as a curved guide (1) along which the carrier member is guided, **characterised in that** the carrier member is constructed from a plurality of link elements (2) arranged movably relative to one another and **in that**, at least outside the guide (1), the link elements (2) are stabilised relative to one another and **in that** the carrier member overall in the stabilised state of the link elements (2) relative to one another is guided non-rotatably relative to the guide (1).

2. A device according to claim 1, **characterised in that** the carrier member is constructed as a resiliently deformable rod.

3. A device according to claim 1, **characterised in that** the carrier member is constructed as a resiliently deformable spring steel.

4. A device according to any one of claims 1 to 3, **characterised in that** the tool (9) is constructed for carrying out mechanical machining.

5. A device according to claims 1 to 4, **characterised in that** the tool (9) is connected to a drive motor.

6. A device according to any one of claims 1 to 5, **characterised in that** a drive shaft for the tool (9) extends along the guide.

7. A device according to any one of claims 1 to 6, **characterised in that** a drive shaft for the tool (9) extends within a tubular envelope.

8. A device according to any one of claims 1 to 7, **characterised in that** the carrier member is coupled with a feed device.

9. A device according to claim 8, **characterised in that** the feed device is constructed for introducing tensile and compressive forces.

10. A device according to any one of claims 1 to 9, **characterised in that** the device is constructed for use in the medical field.

11. A device according to any one of claims 1 to 10, **characterised in that** the tool (9) is constructed for producing cavities for endoprostheses.

12. A device according to claim 11, **characterised in that** an embodiment is provided for producing cavities for hip joint endoprostheses.

13. A device according to any one of claims 1 to 12, **characterised in that** the feed device comprises a linear drive.

14. A device according to any one of claims 1 to 13, **characterised in that** the link elements (2) are tensioned relative to one another by a spring steel wire.

## Revendications

1. Dispositif destiné au positionnement d'un outil (9) pour le traitement d'os, qui présente un manche (3), qui est relié, dans la région de l'une de ses extrémités (5) à l'outil (9), par l'intermédiaire d'un élément de support réversiblement déformable, et dans lequel est prévue une section, qui, disposée à une distance de cette extrémité (5, est destinée à introduire des forces de positionnement, et dans lequel le manche (3) est réalisé, au moins par sections, sous la forme d'un organe de guidage courbé (1), le long duquel est conduit l'élément de support, **caractérisé en ce que** l'élément de support est composé de plusieurs éléments articulés (2) agencés mobiles les uns par rapport aux autres, et que les éléments articulés (2) sont stabilisés les uns par rapport aux autres, au moins en dehors de l'organe de guidage (1), et que l'élément de support, quand les éléments articulés (2) sont stabilisés les uns par rapport aux autres, est guidé en étant empêché de tourner par rapport à l'organe de guidage (1).

2. Dispositif selon la revendication 1, **caractérisé en ce que** l'élément de support est conçu sous la forme d'une barre élastiquement déformable.

3. Dispositif selon la revendication 1, **caractérisé en ce que** l'élément de support est réalisé en acier à ressorts élastiquement déformable.

4. Dispositif selon l'une des revendications 1 à 3, **caractérisé en ce que** l'outil (9) est conçu pour l'exécution de traitements mécaniques.

5. Dispositif selon l'une des revendications 1 à 4, **caractérisé en ce que** l'outil (9) est relié à un moteur de commande.

6. Dispositif selon l'une des revendications 1 à 5, **caractérisé en ce qu'**un arbre de commande, prévu pour l'outil (9), s'étend le long de l'organe de guidage.

7. Dispositif selon l'une des revendications 1 à 6, **caractérisé en ce que** l'arbre de commande, prévu pour l'outil (9) s'étend à l'intérieur d'une enveloppe en forme de tube.

8. Dispositif selon l'une des revendications 1 à 7, **caractérisé en ce que** l'élément de support est couplé avec un dispositif d'avance.

9. Dispositif selon la revendication 8, **caractérisé en ce que** le dispositif d'avance est conçu pour l'application de forces de traction et de pression.

10. Dispositif selon l'une des revendications 1 à 9, **caractérisé en ce que** le dispositif est conçu pour la mise en oeuvre dans le domaine médical.

11. Dispositif selon l'une des revendications 1 à 10, **caractérisé en ce que** l'outil est conçu pour fabrications de cavités pour des endoprothèses.

12. Dispositif selon la revendication 11, **caractérisé en ce que** existe un mode de réalisation pour former des cavités pour des endoprothèses de l'articulation de la hanche.

13. Dispositif selon l'une des revendications 1 à 12, **caractérisé en ce que** le dispositif d'avance présente un entraînement linéaire.

14. Dispositif selon l'une des revendications 1 à 13, **caractérisé en ce que** les éléments articulés (2) sont contraints par rapport les uns aux autres par un fil d'acier à ressorts.
